# FASCICULE DE BREVET EUROPEEN

(11) **EP 4 288 399 B1**
(45) Date de publication et mention de la délivrance du brevet: **19.03.2025**
(21) Numéro de dépôt: 21819928.9
(22) Date de dépôt: 15.11.2021
(51) Int. Cl.: C07C 4/04, C10L 3/10, C10G 9/00, C01B 3/24, C07C 7/00, C07C 7/148

(54) **PROCEDE DE PURIFICATION D'UN FLUX DE GAZ NATUREL**
VERFAHREN ZUR REINIGUNG EINES ERDGASSTROMS
METHOD FOR PURIFYING A FLOW OF NATURAL GAS

(30) Priorité: 05.02.2021 FR 2101124
(43) Date de publication de la demande: 13.12.2023
(73) Titulaire: TOTALENERGIES ONETECH, 92400 Courbevoie (FR)
(72) Inventeur: CALLU, Cyrille, 69360 SOLAIZE (FR); FRANCKE, Loïc, 64018 PAU CEDEX (FR)
(74) Mandataire: Casalonga
(86) Numéro de dépôt international: PCT/FR2021/052014
(87) Numéro de publication internationale: WO 2022/167732

(56) Documents cités:
- EP-A1- 1 288 182
- WO-A1-2018/055264

## Description

La présente invention a pour objet un procédé permettant de purifier un flux de gaz naturel, afin de réduire sa teneur en hydrocarbures indésirables tout en augmentant sa teneur en méthane et en dihydrogène.

Le procédé selon l'invention est particulièrement utile pour préparer des carburants à base de gaz naturel, à haut pouvoir énergétique et faibles niveaux d'émissions.

### ETAT DE L'ART ANTERIEUR

Le gaz naturel est essentiellement constitué d'hydrocarbures légers en C₁ à C₅, parmi lesquels se trouvent une proportion majoritaire de méthane (en général plus de 80% en moles, voire plus de 90% en moles), et une proportion minoritaire d'hydrocarbures supérieurs tels qu'en particulier de l'éthane, du propane, des butanes et des pentanes, dont la teneur diminue avec l'augmentation du nombre d'atomes de carbone. Ainsi par exemple la teneur en éthane du gaz naturel peut aller jusqu'à 10% en moles, la teneur en propane jusqu'à 1% en moles, la teneur en butanes jusqu'à 0,3% en moles.

Le gaz naturel peut également comprendre d'autres composés différents des hydrocarbures, en proportion très mineure. Selon sa provenance le gaz naturel peut ainsi par exemple comprendre, en très faible teneur, de l'azote, du dioxyde de carbone, du dihydrogène, du sulfure d'hydrogène, de l'hélium, des mercaptans.

Le gaz naturel est de plus en plus utilisé comme carburant pour alimenter des moteurs présents tant dans des véhicules individuels que des véhicules spéciaux tels que par exemple des camions, des bus, des bennes à ordures...

Afin d'une part d'augmenter le rendement énergétique des moteurs fonctionnant au gaz naturel, et d'autre part de diminuer les émissions polluantes (notamment de dioxyde de carbone) de ces moteurs, les gaz naturels utilisés doivent avoir une teneur la plus importante possible en méthane, et des teneurs aussi faibles que possibles en hydrocarbures supérieurs comprenant au moins deux atomes de carbone (ci-après dénommés hydrocarbures en C₂₊). Le document WO 2018/055264 porte sur un procédé de purification de gaz naturel à liquéfier, où le gaz naturel est entre autres soumis à une distillation fractionnée.

### OBJET DE L'INVENTION

La présente invention vise à proposer un procédé original de purification du gaz naturel, qui permet d'atteindre les objectifs ci-dessus de réduction de sa teneur en hydrocarbures en C₂₊, tout en augmentant parallèlement sa teneur en méthane.

De manière particulièrement avantageuse, le procédé de l'invention permet en outre d'enrichir le gaz naturel en dihydrogène, sans pour autant générer de dioxyde de carbone.

La présente invention a ainsi pour objet un procédé de purification d'un flux de gaz naturel, qui comprend les étapes suivantes:
a) une étape de traitement d'au moins une partie dudit flux par pyrolyse à une température comprise dans la gamme allant de 1000°C à 2000°C, de manière à décomposer les hydrocarbures comprenant au moins deux atomes de carbone en carbone élémentaire et en dihydrogène H₂ et à obtenir ainsi un flux traité, puis
b) une étape d'élimination du carbone élémentaire présent dans le flux traité issu de l'étape a) de manière à obtenir un flux traité exempt de carbone élémentaire; puis
c) lorsque les étapes a) et b) ont été effectuées sur une partie seulement du flux de gaz naturel, une étape de mélange du flux traité exempt de carbone élémentaire issu de l'étape b) avec la partie du flux non traitée ; puis
d) l'obtention d'un flux de gaz naturel purifié constitué soit du mélange issu de l'étape c) soit du flux traité exempt de carbone élémentaire issu de l'étape b).

Le procédé selon l'invention consiste ainsi à faire subir, à tout ou partie du flux de gaz naturel, un traitement qui permet d'éliminer sélectivement les hydrocarbures en C₂₊ présents tels que l'éthane, le propane, le butane, le pentane, en les transformant par pyrolyse à haute température en carbone élémentaire et en dihydrogène, puis en éliminant le carbone élémentaire résultant de la pyrolyse.

Ainsi, du fait de l'élimination des hydrocarbures en C₂₊, la teneur pondérale en méthane du gaz naturel augmente.

L'invention permet ainsi d'augmenter l'indice de méthane du flux de gaz naturel.

Elle permet également de contrôler l'indice de méthane du gaz naturel, et de garantir l'obtention d'un gaz naturel traité ayant un indice de méthane constant dans une gamme requise, ou supérieur à un seuil minimal, satisfaisant par exemple à une spécification.

De manière connue en soi, l'indice de méthane d'un gaz combustible est un paramètre numérique compris dans la gamme de 0 à 100, et qui correspond à la mesure de la résistance du gaz au cliquetis dans le cadre d'un essai de combustion normalisé conformément à la norme ISO/TR 22302 :2014. Le méthane pur, utilisé comme combustible de référence, possède un indice de méthane de 100.

Dans le cas où le gaz naturel est destiné à être utilisé comme base d'un carburant moteur, il est essentiel qu'il ait un indice de méthane constant, afin de garantir les performances du moteur. Par exemple, la spécification EN 16732-2 exige un indice de méthane supérieur à 60, mais les constructeurs automobiles recommandent un indice de méthane supérieur à 70 (voir annexe D de la même norme).

D'une manière générale, on cherche à obtenir un carburant ayant un indice de méthane le plus élevé possible.

La présente invention permet ainsi par exemple d'augmenter l'indice du méthane de la partie du flux traité d'une valeur inférieure à 70 à une valeur supérieure à 80.

De plus, le procédé selon l'invention a pour effet de produire du dihydrogène (H₂) et donc d'en augmenter la teneur en H₂ dans le flux de gaz naturel. Ce dihydrogène dit « dihydrogène turquoise » est ainsi produit, sans émission de dioxyde de carbone.

Lorsque le gaz naturel est destiné à être utilisé comme carburant moteur, une augmentation de sa teneur en dihydrogène est bénéfique car elle diminue les émissions de dioxyde de carbone par diminution de l'intensité en carbone du carburant mais aussi par l'augmentation du rendement moteur (donc une diminution supplémentaire des émissions de CO2 à l'échappement).

En augmentant la teneur en dihydrogène d'un carburant à base de gaz naturel, l'on observe également une diminution des émissions de particules à l'échappement, ainsi qu'une diminution des émissions de monoxyde de carbone (CO) et d'hydrocarbures dont le méthane (CH₄).

Le procédé selon l'invention est particulièrement avantageux dans la production de gaz naturel destiné à une utilisation comme carburant dans un moteur à combustion interne.

Ainsi, la présente invention a également pour objet un procédé de préparation d'un carburant pour moteur à combustion interne, comprenant :
(i) la purification d'un flux de gaz naturel au moyen du procédé selon l'invention ; puis
(ii) le conditionnement du flux de gaz naturel issu de l'étape (i) sous forme de gaz naturel comprimé, de gaz naturel adsorbé ou de gaz naturel liquéfié, de préférence sous forme de gaz naturel comprimé.

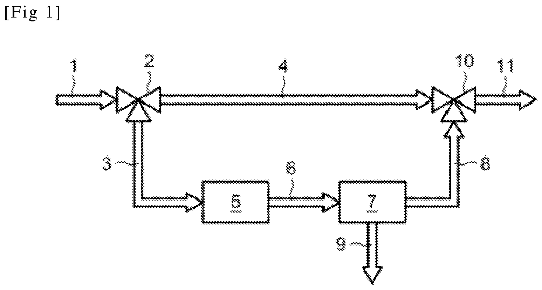

D'autres objets, caractéristiques, aspects et avantages de l'invention apparaîtront encore plus clairement à la lecture de la description et des exemples qui suivent, et au vu de la Figure 1 ci-jointe.

### [Fig 1]

La Figure 1 est une représentation schématique du procédé selon l'invention.

Dans ce qui va suivre, et à moins d'une autre indication, les bornes d'un domaine de valeurs sont comprises dans ce domaine, notamment dans les expressions « compris entre » et « allant de ... à ...».

Par ailleurs, les expressions « au moins un » et « au moins » utilisées dans la présente description sont respectivement équivalentes aux expressions « un ou plusieurs » et « supérieur ou égal ». Enfin, de manière connue en soi, on désigne par composé ou groupe en C_{N} un composé ou un groupe contenant dans sa structure chimique N atomes de carbone.

### DESCRIPTION DETAILLEE

Le procédé selon l'invention est schématiquement représenté dans la Figure 1, dans laquelle un flux de gaz naturel 1 est divisé en deux parties : une première partie 3, acheminée vers l'unité 5 dans laquelle elle est traitée par pyrolyse tandis que l'autre partie 4 du flux 1 de gaz initial, n'est pas traitée. Une vanne à trois voies 2 permet de réguler la proportion du flux de gaz naturel soumise au traitement.

Ainsi, selon un premier mode opératoire, il est possible de fermer la vanne 2, de telle sorte que l'intégralité du flux 1 de gaz naturel est acheminé vers l'unité de pyrolyse 5 et que le flux de gaz non traité 4 est nul.

Selon un second mode opératoire, la vanne 2 est ouverte en partie, de telle sorte qu'une partie 3 du flux de gaz naturel 1 est acheminé vers l'étape de pyrolyse 5.

De préférence, la proportion du flux de gaz naturel 1 soumise aux étapes a) et b) du procédé selon l'invention (c'est-à-dire la partie 3 du flux 1 de gaz initial acheminé vers l'étape de pyrolyse 5) représente au moins 10% en masse du flux 1 de gaz initial. De préférence, cette partie représente de 10 à 100% en masse du flux initial de gaz naturel, de préférence de 20 à 80% en masse.

Cette proportion peut être ajustée en fonction de la composition du flux de gaz naturel initial (et notamment en fonction de sa teneur en hydrocarbures C₂₊) et des spécifications souhaitées pour le gaz naturel purifié, en faisant varier l'ouverture de la vanne 2.

La partie 3 du flux de gaz naturel soumise aux étapes a) et b) du procédé est soumise à une étape 5 de traitement par pyrolyse 5 à une température comprise dans la gamme allant de 1000°C à 2000°C, de manière à décomposer les hydrocarbures en C₂₊ en carbone élémentaire et en dihydrogène (H₂).

Le flux traité 6 issu de l'étape 5 de pyrolyse subit une étape 7 dans laquelle le carbone élémentaire généré à l'étape 5 est éliminé, et évacué. Un flux de carbone élémentaire 9 est ainsi récupéré, et peut être valorisé.

Le carbone ainsi récupéré peut être valorisé sous forme de noir de carbone (qui est une matière première utile notamment dans les encres et les pneumatiques), ou lorsque la méthode de pyrolyse employée conduit à des formes structurales spécifiques du carbone, sous forme de graphite, de graphène, ou d'autres formes

Le flux de gaz traité 8 débarrassé du carbone élémentaire issu de l'étape 7 est enfin mélangé à la partie 4 du flux non traitée dans le cas du second mode opératoire décrit ci-dessus. Une vanne à trois voies 10 permet de réguler les proportions du mélange des deux parties de flux, à savoir la partie traitée 8 débarrassé du carbone élémentaire et la partie non traitée 4, en fonction notamment de leurs compositions respectives et des spécifications requises pour le flux de gaz naturel purifié final 11 résultant de ce mélange. Des régulateurs de pression (non représentés) peuvent être avantageusement positionnés en amont de la vanne à trois voies 10 afin d'assurer la même pression pour le flux 8 et le flux 4.

### L'étape a) de traitement par pyrolyse

L'étape a) du procédé selon l'invention consiste à traiter tout ou partie du flux de gaz naturel dans une unité de pyrolyse.

La pyrolyse est un traitement bien connu de l'homme du métier, qui consiste à porter un produit à une température très élevée, de manière à provoquer sa décomposition thermique.

Dans la présente invention, il est essentiel de contrôler la température de l'étape de pyrolyse et de la maintenir dans une gamme allant de 1000°C à 2000°C. A ces températures, les hydrocarbures présents dans le flux de gaz naturel se décomposent en carbone élémentaire C et en dihydrogène H₂. Dans cette gamme de températures, les hydrocarbures ont une cinétique de décomposition thermique qui augmente avec leur nombre d'atomes de carbone, ce qui signifie que les hydrocarbures les plus lourds (ie ayant les chaînes carbonées les plus longues) sont les premiers à être décomposés. Ainsi, à une température donnée, compte tenu de la cinétique de décomposition des différents hydrocarbures présents, en contrôlant le temps de séjour du flux de gaz dans l'unité de pyrolyse, il est possible de décomposer les hydrocarbures en C₂₊, sans dégrader de manière conséquente le méthane. Il est inévitable qu'une proportion de méthane soit également décomposée, mais les conditions opératoires de l'étape a), et notamment la température de pyrolyse et le temps de séjour du flux dans l'unité de pyrolyse, peuvent être aisément ajustés par l'homme du métier de manière à maximiser la décomposition des hydrocarbures en C₂₊ et minimiser celle du méthane. Avantageusement, le temps de séjour du flux dans l'unité de pyrolyse est compris dans la gamme allant de 0,01s à 1s.

Le temps de séjour du flux de gaz traité dans l'unité de pyrolyse peut être contrôlé en ajustant son débit.

De préférence, la température de pyrolyse à l'étape a) est comprise dans la gamme allant de 1100 à 1600°C, de préférence de 1200 à 1500°C.

La réalisation de l'étape a) n'est pas limitée à une technique de pyrolyse spécifique, et toutes les techniques de pyrolyse connues peuvent être utilisées du moment qu'elles permettent d'atteindre les températures requises et de contrôler de manière précise le temps de séjour du flux ou de la partie de flux de gaz naturel traité à l'étape a), de manière à maximiser la décomposition sélective des hydrocarbures en C₂₊.

Selon un premier mode de réalisation, le traitement de pyrolyse est effectué en chauffant le flux ou la partie de flux de gaz naturel au moyen d'un plasma chaud.

Le plasma chaud peut être généré en utilisant différentes technologies, connues de l'homme du métier, telles que notamment par micro-ondes, par torche à plasma, ou par courant alternatif.

La technologie par micro-ondes consiste à utiliser un ou plusieurs modules de génération de micro-ondes standards (athermique) avec un couplage micro-onde/gaz dans un réacteur, de manière à produire un plasma chaud à partir du gaz naturel. Ce plasma est initié et entretenu par le transfert des électrons dus aux micro-ondes. Il est possible d'utiliser plusieurs modules en série, suivant le taux de conversion voulu.

La technologie par torche à plasma permet d'introduire des jets de plasma dans la chambre où passe le gaz naturel. La température de la torche (généralement supérieure ou égale à 6000°C) permet de porter la chambre à la température requise pour effectuer la pyrolyse sélective du gaz naturel.

La technologie de courant alternatif consiste à générer de la chaleur en faisant passer un courant alternatif dans le réacteur dans lequel circule le flux de gaz naturel traité. Le courant alternatif crée des arcs électriques dans le réacteur. C'est l'énergie de ces arcs qui échauffe le gaz naturel, le fait passer à l'état de plasma, et permet sa décomposition.

Selon un second mode de réalisation, le traitement de pyrolyse est effectué en faisant circuler le flux ou la partie de flux de gaz naturel dans une colonne remplie de métal fondu. On peut employer à cet effet tout métal approprié, ayant un point de fusion dans la gamme de température requise, tel que par exemple l'indium (In), le gallium (Ga), le bismuth (Bi), l'étain (Sn), le plomb (Pb) et le nickel (Ni).

Selon un troisième mode de réalisation, le traitement de pyrolyse est effectué en chauffant le flux ou la partie de flux de gaz naturel par ondes de choc.

Cette technologie consiste à utiliser l'énergie cinétique d'un train d'onde, créé soit par des différences de pression soit par une valve électromagnétique à haute pression, dans un tube à chocs pour échauffer le gaz naturel à la température requise. Le système peut être optimisé en utilisant un rotor pour améliorer les rendements de conversion.

Dans le cadre d'un procédé en boucle ouverte (configuration dite « open loop »), le gaz naturel arrive sous pression (70 bars) et le flux sortant est aux environs de 30 bars. Pour améliorer le taux de conversion, il est possible d'utiliser un procédé en boucle fermée (configuration dite « closed loop »), qui permet de faire recirculer une partie du flux de gaz naturel traité pour augmenter les taux de conversion du gaz à traiter.

Selon un quatrième mode de réalisation, le traitement de pyrolyse est effectué en chauffant le flux ou la partie de flux de gaz naturel par induction, soit de manière directe soit de manière indirecte par exemple via un catalyseur (métallique ou carboné).

Quelle que soit la technologie de pyrolyse mise en œuvre, il est possible de mettre en œuvre plusieurs unités (ou réacteurs) de traitement en série, et/ou de recycler une partie du flux de gaz traité vers l'unité ou le réacteur de pyrolyse, pour atteindre le taux souhaité de décomposition des hydrocarbures en C₂₊.

A l'issue de l'étape a), un flux traité contenant une proportion importante de méthane, du carbone élémentaire généralement à l'état solide, et de dihydrogène est obtenu.

La teneur du flux traité en hydrocarbures en C₂₊ est généralement inférieure à 0,5% en moles, de préférence inférieure à 0,1% et mieux encore égale à 0% en moles.

### L'étape b) d'élimination du carbone

L'étape b) du procédé selon l'invention consiste à éliminer le carbone élémentaire du flux de gaz naturel traité issu de l'étape a).

Différentes technologies peuvent être utilisées à cet effet, et l'invention n'est pas limitée à l'utilisation d'une technologie particulière.

Selon un premier mode de réalisation, l'étape b) est réalisée en en faisant circuler le flux de gaz naturel traité issu de l'étape a) dans un bain de sel fondu.

Ce bain de sel fondu permet de piéger le carbone qui se solidifie dans le bain, et de récupérer ainsi un flux de gaz débarrassé du carbone élémentaire. Le carbone solide remonte en surface du bain de sel fondu, duquel il est ensuite régulièrement éliminé de manière mécanique.

Selon un second mode de réalisation, l'étape b) est réalisée en refroidissant le flux de gaz naturel traité issu de l'étape a) (par exemple au moyen d'un échangeur de chaleur, par exemple alimenté en eau froide) puis en le faisant circuler dans un ou plusieurs dispositifs de séparation mécanique tels que notamment un ou plusieurs cyclones, un ou plusieurs filtres.

Ces types de dispositifs, connus en soi, permettent de récupérer le carbone sous forme de particules solides. Il peut être avantageux de mettre en œuvre plusieurs cyclones et/ou plusieurs filtres, disposés en série.

Une variante particulièrement préférée de ce mode de mise en œuvre consiste à faire circuler ledit flux de gaz naturel traité dans des dispositifs de séparation mécanique comprenant un ou plusieurs cyclones, suivis d'un ou plusieurs filtres. Le cyclone permet de faire coalescer les particules afin d'améliorer l'efficacité de la filtration.

A l'issue de l'étape b), un flux exempt de carbone élémentaire, et enrichi en dihydrogène, tout en contenant une proportion importante de méthane, est obtenu. Ce flux issu de l'étape b) est également exempt d'hydrocarbures en C2+. Il contient typiquement une quantité de dihydrogène allant de 10 à 30% en moles, de préférence de 10 à 20% en moles.

Par exempt de carbone élémentaire, on désigne un flux dont la teneur en carbone élémentaire est inférieure à 0,1% en moles.

Par exempt d'hydrocarbures en C2+, on désigne un flux dont la teneur en hydrocarbures en C2+ est inférieure à 0,5% en moles.

### Le procédé de préparation de carburant

Selon un mode de réalisation avantageux de l'invention, le flux de gaz naturel purifié au moyen du procédé décrit ci-avant est ensuite utilisé pour préparer un carburant pour moteur à combustion interne.

Le carburant ainsi obtenu peut se présenter sous forme de gaz naturel comprimé (GNC, à pression d'environ 200 bars), de gaz naturel adsorbé (ANG) ou de gaz naturel liquéfié (GNL, à température d'environ -160°C). De préférence, il se présente sous forme de gaz naturel comprimé, c'est-à-dire maintenu sous une forte pression, typiquement de 200 bars (2.10⁷ Pa)

Le carburant peut également comprendre un ou plusieurs additifs, qui peuvent être choisis parmi tous les additifs usuellement employés dans la formulation de carburants à base de gaz naturel.

On peut citer par exemple les additifs réodorants, qui incluent notamment des composés soufrés (tétrahydrothiophène (THT) ou méthylmercaptan (ou méthanethiol)), et peuvent être présents dans des teneurs allant notamment de 15 à 40 mg/m³.

L'exemple ci-après vise simplement à illustrer l'invention sans en limiter la portée.

### EXEMPLE

Le flux de gaz naturel mis en œuvre dans cet exemple présente la composition détaillée dans le tableau 1 ci-dessous.

**[Tableau 1]**

| Composé | Teneur (en % en moles) |
|---|---|
| Azote N₂ | 0,423 |
| Méthane CH₄ | 90,678 |
| Ethane C₂H₆ | 7,949 |
| Propane C₃H₈ | 0,743 |
| Iso-butane iC₄H₁₀ | 0,074 |
| Normal-butane nC₄H₁₀ | 0,112 |
| Iso-pentane iC₃H₁₂ | 0,016 |
| Normal-pentane nC₅H₁₂ | 0,006 |

Ce flux de gaz naturel présente un débit de 220 tonnes/jour, et est traité conformément au procédé de l'invention, de la manière suivante :
a) tout ou partie du flux de gaz est traité par pyrolyse, dans un réacteur à plasma à micro-ondes, constitué d'un tube d'injection autour duquel un guide d'onde apporte les micro-ondes qui créent un plasma au sein du tube d'injection, permettant ainsi la pyrolyse. Les micro-ondes sont créées par un magnétron, mais on peut également employer un générateur de micro-onde à semi-conducteurs. La température au sein du tube d'injection est maintenue dans la gamme de 1200 à 1500°C. Dans ce réacteur, l'intégralité des hydrocarbures en C₂ à C₅ et environ 5% du méthane sont décomposés en carbone élémentaire C et dihydrogène H₂. Les conditions opératoires choisies conduisent ainsi à une forte sélectivité en faveur de la conversion des hydrocarbures en C₂₊ ;
b) le flux traité sortant du réacteur à plasma circule ensuite dans une unité de séparation contenant un cyclone suivi d'un filtre de manière à séparer le carbone sous forme de particules solides et récupérer ainsi un flux exempt de carbone élémentaire et d'hydrocarbures en C2+, et enrichi en dihydrogène. Le flux traité entre dans le cyclone à une température d'environ 250°C et en ressort à une température de 50°C.

On fait varier la proportion en masse du flux de gaz soumis aux étapes a) et b) ci-dessous.

Dans un premier test, l'intégralité du flux de gaz est traitée (proportion de 100%).

Dans les tests suivants, on ne traite qu'une partie du flux de gaz, en faisant varier la proportion du flux de gaz traité de 20 à 80% en masse, et on mélange ensuite cette partie du flux traitée et débarrassé du carbone élémentaire issue des étapes a) et b) à la partie du flux initial non traitée.

Les quantités des différents composés présents dans le flux de gaz naturel purifié final et la quantité de carbone élémentaire C récupéré sont détaillés dans le tableau 2 ci-dessous, pour chacun des essais.

**[Tableau 2]**

| Proportion du flux de gaz traité | Débit de H₂ (tonnes/jour) | Débit de C (tonnes/jour) | Débit de CH₄ (tonnes/jour) |
|---|---|---|---|
| 100% | 5,8 | 21,1 | 192,2 |
| 80% | 4,6 | 16,9 | 193,8 |
| 60% | 3,5 | 12,6 | 195,3 |
| 40% | 2,3 | 8,4 | 196,9 |
| 20% | 1,2 | 4,2 | 198,4 |

Le tableau 3 ci-dessous indique la teneur molaire en dihydrogène H₂ dans le flux de gaz naturel purifié final.

**[Tableau 3]**

| Proportion du flux de gaz traité | Teneur en H₂ du flux de gaz naturel purifié (% en moles) |
|---|---|
| 100% | 24,1% |
| 80% | 19,1% |
| 60% | 14,2% |
| 40% | 9,4% |
| 20% | 4,7% |

## Revendications

1. Procédé de purification d'un flux (1) de gaz naturel, comprenant les étapes suivantes:
a) une étape (5) de traitement d'au moins une partie (3) dudit flux (1) par pyrolyse à une température comprise dans la gamme allant de 1000°C à 2000°C, de manière à décomposer les hydrocarbures comprenant au moins deux atomes de carbone en carbone élémentaire et en dihydrogène H₂ et à obtenir ainsi un flux traité (6), puis
b) une étape (7) d'élimination du carbone élémentaire présent dans le flux traité (6) issu de l'étape a) de manière à obtenir un flux traité exempt de carbone élémentaire (8) ; puis
c) lorsque les étapes a) et b) ont été effectuées sur une partie seulement du flux (1) de gaz naturel, une étape (10) de mélange du flux traité exempt de carbone élémentaire (8) issu de l'étape b) avec la partie (4) du flux non traité ; puis
d) l'obtention d'un flux de gaz naturel purifié (11) constitué soit du mélange (10) issu de l'étape c) soit du flux traité exempt de carbone élémentaire (8) issu de l'étape b).

2. Procédé selon la revendication précédente, **caractérisé en ce que** la température de pyrolyse à l'étape a) est comprise dans la gamme allant de 1100 à 1600°C, de préférence de 1200 à 1500°C.

3. Procédé selon l'une quelconque des revendications précédentes, **caractérisé en ce que** le traitement de pyrolyse est effectué en chauffant le flux ou la partie de flux de gaz naturel au moyen d'un plasma chaud.

4. Procédé selon la revendication précédente, **caractérisé en ce que** le plasma chaud est généré par micro-ondes, par torche à plasma, ou par courant alternatif.

5. Procédé selon l'une quelconque des revendications 1 et 2, **caractérisé en ce que** le traitement de pyrolyse est effectué en faisant circuler le flux ou la partie de flux de gaz naturel dans une colonne remplie de métal fondu.

6. Procédé selon l'une quelconque des revendications 1 et 2, **caractérisé en ce que** le traitement de pyrolyse est effectué en chauffant le flux ou la partie de flux de gaz naturel par ondes de choc.

7. Procédé selon l'une quelconque des revendications 1 et 2, **caractérisé en ce que** le traitement de pyrolyse est effectué en chauffant le flux ou la partie de flux de gaz naturel par induction, soit de manière directe soit de manière indirecte par exemple via un catalyseur.

8. Procédé selon l'une quelconque des revendications précédentes, **caractérisé en ce que** la proportion du flux de gaz naturel (1) soumise aux étapes a) et b) représente au moins 10% en masse du flux (1) de gaz, de préférence de 10 à 100% en masse dudit flux (1), plus préférentiellement de 20 à 80% en masse.

9. Procédé selon l'une quelconque des revendications précédentes, **caractérisé en ce que** l'étape b) est réalisée en faisant circuler le flux de gaz naturel traité issu de l'étape a) dans un bain de de sel fondu.

10. Procédé selon l'une quelconque des revendications 1 à 8, **caractérisé en ce que** l'étape b) est réalisée en refroidissant le flux de gaz naturel traité issu de l'étape a) puis en le faisant circuler dans un ou plusieurs dispositifs de séparation mécanique.

11. Procédé selon la revendication précédente, **caractérisé en ce que** les dispositifs de séparation mécanique comprennent un ou plusieurs cyclones, suivis d'un ou plusieurs filtres.

12. Procédé de préparation d'un carburant pour moteur à combustion interne, comprenant :
(i) la purification d'un flux de gaz naturel au moyen du procédé tel que défini dans l'une quelconque des revendications précédentes ; puis
(ii) le conditionnement du flux de gaz naturel issu de l'étape (i) sous forme de gaz naturel comprimé, de gaz naturel adsorbé ou de gaz naturel liquéfié, de préférence sous forme de gaz naturel comprimé.

## Patentansprüche

1. Verfahren zum Reinigen eines Erdgasstroms (1), das die folgenden Schritte umfasst:
a) einen Schritt (5) der Behandlung von mindestens einem Teil (3) des Stroms (1) durch Pyrolyse bei einer Temperatur im Bereich von 1000 °C bis 2000 °C, so dass die Kohlenwasserstoffe, die mindestens zwei Kohlenstoffatome umfassen, in elementaren Kohlenstoff und Diwasserstoff H₂ zersetzt werden und so ein behandelter Strom (6) erhalten wird, dann
b) einen Schritt (7) des Entfernens des in dem behandelten Strom (6) aus Schritt a) vorhandenen elementaren Kohlenstoffs, um einen behandelten Strom (8) ohne elementarem Kohlenstoff zu erhalten; dann
c) wenn die Schritte a) und b) nur an einem Teil des Erdgasstroms (1) durchgeführt wurden, einen Schritt (10) des Mischens des behandelten Stroms (8) ohne elementaren Kohlenstoff aus Schritt b) mit dem Teil (4) des unbehandelten Stroms; dann
d) Erhalten eines gereinigten Erdgasstroms (11), der entweder aus dem Gemisch (10) aus Schritt c) oder aus dem behandelten Strom (8) ohne elementarem Kohlenstoff aus Schritt b) besteht.

2. Verfahren nach dem vorhergehenden Anspruch, **dadurch gekennzeichnet, dass** die Pyrolysetemperatur in Schritt a) im Bereich von 1100 bis 1600 °C, vorzugsweise von 1200 bis 1500 °C, liegt.

3. Verfahren nach einem der vorhergehenden Ansprüche, **dadurch gekennzeichnet, dass** die Pyrolysebehandlung durch Erhitzen des Erdgasstroms oder des Teils des Erdgasstroms mittels eines heißen Plasmas erfolgt.

4. Verfahren nach dem vorhergehenden Anspruch, **dadurch gekennzeichnet, dass** das heiße Plasma durch Mikrowellen, einen Plasmabrenner oder Wechselstrom erzeugt wird.

5. Verfahren nach einem der Ansprüche 1 und 2, **dadurch gekennzeichnet, dass** die Pyrolysebehandlung durch Zirkulation des Erdgasstroms oder des Erdgasstromteils in einer mit geschmolzenem Metall gefüllten Säule erfolgt.

6. Verfahren nach einem der Ansprüche 1 und 2, **dadurch gekennzeichnet, dass** die Pyrolysebehandlung durch Erhitzen des Erdgasstroms oder des Erdgasstromteils durch Stoßwellen erfolgt.

7. Verfahren nach einem der Ansprüche 1 und 2, **dadurch gekennzeichnet, dass** die Pyrolysebehandlung durch Erhitzen des Erdgasstroms oder des Erdgasstromteils durch Induktion entweder direkt oder indirekt beispielsweise über einen Katalysator erfolgt.

8. Verfahren nach einem der vorhergehenden Ansprüche, **dadurch gekennzeichnet, dass** der Anteil des Erdgasstroms (1), der den Schritten a) und b) unterliegt, mindestens 10 Masse-% des Gasstroms (1), vorzugsweise 10 bis 100 Masse-% des Gasstroms (1), vorzugsweise 20 bis 80 Masse-%, ausmacht.

9. Verfahren nach einem der vorhergehenden Ansprüche, **dadurch gekennzeichnet, dass** der Schritt b) durch Zirkulation des behandelten Erdgasstroms aus Schritt a) in einem Bad aus geschmolzenem Salz durchgeführt wird.

10. Verfahren nach einem der Ansprüche 1 bis 8, **dadurch gekennzeichnet, dass** der Schritt b) durch Kühlen des aus dem Schritt a) stammenden behandelten Erdgasstroms und anschließendes Zirkulieren in einer oder mehreren mechanischen Trennvorrichtungen durchgeführt wird.

11. Verfahren nach dem vorhergehenden Anspruch, **dadurch gekennzeichnet, dass** die mechanischen Trennvorrichtungen einen oder mehrere Zyklone, gefolgt von einem oder mehreren Filtern, umfassen.

12. Verfahren zum Herstellen eines Kraftstoffs für Verbrennungsmotoren, das Folgendes umfasst:
(i) Reinigen eines Erdgasstroms durch das Verfahren gemäß einem der vorhergehenden Ansprüche; dann (ii) das Konditionieren des Erdgasstroms aus dem Schritt (i) in Form von komprimiertem Erdgas, adsorbiertem Erdgas oder Flüssigerdgas, vorzugsweise in Form von komprimiertem Erdgas.

## Claims

1. A method for purifying a flow (1) of natural gas, comprising the following steps:
a) a step (5) of processing at least a portion (3) of said flow (1) by pyrolysis at a temperature in the range of from 1000°C to 2000°C, so as to decompose hydrocarbons comprising at least two carbon atoms into elemental carbon and dihydrogen H₂ and thus obtain a processed flow (6), then
b) a step (7) of eliminating the elemental carbon present in the processed flow (6) from step a) so as to obtain a processed flow (8) free of elemental carbon; then
c) when steps a) and b) have been carried out on a portion of the flow (1) of natural gas only, a step (10) of mixing the processed flow (8) free of elemental carbon from step b) with the portion (4) of the non-processed flow; then
d) obtaining a flow of purified natural gas (11) consisting either of the mixture (10) from step c) or of the processed flow (8) free of elemental carbon from step b).

2. The method according to the preceding claim, **characterised in that** the pyrolysis temperature in step a) is in the range of from 1100 to 1600°C, preferably from 1200 to 1500°C.

3. The method according to any of the preceding claims, **characterised in that** the pyrolysis step is carried out by heating the flow or the portion of the flow of natural gas by means of a hot plasma.

4. The method according to the preceding claim, **characterised in that** the hot plasma is generated by microwaves, plasma torch or alternating current.

5. The method according to any of claims 1 and 2, **characterised in that** the pyrolysis step is carried out by circulating the flow or portion of the flow of natural gas in a column filled with molten metal.

6. The method according to any of claims 1 and 2, **characterised in that** the pyrolysis step is carried out by heating the flow or the portion of the flow of natural gas by shock waves.

7. The method according to any of claims 1 and 2, **characterised in that** the pyrolysis step is carried out by heating the flow or the portion of the flow of natural gas by induction, either directly or indirectly, for example via a catalyst.

8. The method according to any of the preceding claims, **characterised in that** the proportion of the flow of natural gas (1) subjected to steps a) and b) accounts for at least 10% by weight of the gas flow (1), preferably from 10 to 100% by weight of said flow (1), more preferably from 20 to 80% by weight.

9. The method according to any of the preceding claims, **characterised in that** step b) is carried out by circulating the processed flow of natural gas from step a) in a molten salt bath.

10. The method according to any of claims 1 to 8, **characterised in that** step b) is carried out by cooling the processed flow of natural gas from step a) and then circulating it in one or more mechanical separation devices.

11. The method according to the preceding claim, **characterised in that** the mechanical separation devices comprise one or more cyclones, followed by one or more filters.

12. A method for preparing a fuel for an internal combustion engine, comprising:
(i) purifying a flow of natural gas using the method as defined in any of the preceding claims; and then
(ii) conditioning the flow of natural gas from step (i) as compressed natural gas, adsorbed natural gas or liquefied natural gas, preferably as compressed natural gas.
